# EUROPEAN PATENT SPECIFICATION

(11) **EP 3 987 336 B1**
(45) Date of publication and mention of the grant of the patent: **23.08.2023**
(21) Application number: 20716119.1
(22) Date of filing: 27.03.2020
(51) Int. Cl.: G02B 25/00, G02B 27/01, G02C 7/08

(54) **DIGITAL MAGNIFIER MONOCLE AND RELATIVE EYEWEAR**
DIGITALES LUPENMONOKEL UND ENTSPRECHENDE BRILLE
MONOCLE À LOUPE NUMÉRIQUE ET LUNETTES RELATIVES

(30) Priority: 04.04.2019 IT 201900005110
(43) Date of publication of application: 27.04.2022
(73) Proprietor: Eye Tech Lab S.r.l., 20121 Milano (IT)
(72) Inventor: MOSCATELLI, Davide, 20121 Milano (IT); COCCOLI, Mauro, 20121 Milano (IT); POZZI, Stefano, 20121 Milano (IT)
(74) Representative: Tana, Maria Gabriella
(86) International application number: PCT/IB2020/052926
(87) International publication number: WO 2020/201955

(56) References cited:
- WO-A1-2012/018784
- WO-A2-2011/002209
- EPO: "computer generated translation of KR20110001952A", , 29 January 2020 (2020-01-29), XP055663346, Retrieved from the Internet: URL:http://tfly.internal.epo.org/index.htm l?num=KR20110001952&type=PN [retrieved on 2020-01-29]

## Description

### TECHNICAL FIELD OF THE INVENTION

The present invention relates to a digital magnifier monocle that can be mounted on magnifying eyewear, or magnifier eyewear, otherwise known in the industry by the term "e loupes". Therefore, the subject of the present invention also includes magnifier eyewear comprising: a frame and two digital magnifier monocles mounted directly on said frame; and magnifier eyewear comprising: a frame, two so-called carrier lenses and two digital magnifier monocles, each of which mounted on a carrier lens.

A monocle according to the present invention comprises, in particular: an optical focusing system, a lens optical system and an assembly of optoelectronic systems mounted between the two optical systems, said assembly being composed of: a camera for the acquisition of the images, a screen for the display of the acquired images and an electronic module suitably designed to ensure the transfer, almost in real time, of the images from the camera to the screen and to allow the interfacing with optional external augmented reality systems or systems for storage and/or processing of the acquired images. A monocle constituted in this way can be used, in particular, in magnifier eyewear, such as that used in the medical or dental field. The possibility of interfacing with systems for augmented reality, in fact, may be of particular interest in the diagnostic or surgical field. In these fields it is of considerable utility to overlay images magnified by the monocle with both alphanumerical information related, for example, to vital parameters (e.g. blood pressure, heart rate, oxygen saturation, etc.), and medical digital images previously acquired with different imaging techniques, such as for example magnetic resonance imaging (MRI) and computed tomography (CT). In the dental field, on the other hand, the use of augmented reality in magnifier eyewear can be relevant in the programming and performance of osseointegrated implants insertion procedures, where the integration of images acquired in real time from the monocle with CT - Dental Scan images can be useful. Finally, integration may also be of interest of the acquired images with diagnostic information obtained through appropriate deep learning algorithms applied to the same images acquired, in real time, by the camera forming part of the monocle. An example of the application of the latter type of integration can be the evaluation of skin melanomas through the display of graphic contouring and a textual indication superimposed on the image itself of the melanoma.

### STATE OF THE ART

As mentioned above, magnifier eyewear, preferably, but not exclusively, for medical and dental purposes, is a product that is extensively widespread in the world. At the present state of the art, two main types of magnifier eyewear of analogical type are known, i.e. equipped with analogue magnifier monocles. The latter can be attached either directly to the frame of the eyewear or to the carrier lenses, attached in turn on the frame. More particular, in the first type, also known by the term "FlipUp", the magnifier monocles are mounted on a support structure that is adjustable and rotatable; in the second type, also known by the expression "Through the Lens", the monocles are, instead, glued inside special holes applied to the carrier lenses. The "Through the Lens" type has considerable optical advantages but needs 100% user customisation; the "FlipUp" type, instead, while allowing the creation of eyewear adaptable to different people, is, without doubt, less comfortable.

The general purpose of magnifier eyewear is to enlarge a portion of the field of vision in which operations are being performed, at a working distance, usually fixed, bringing this portion in the user's field of vision and in the natural position of near vision. The high precision of the positioning, even more so if customised for each operator, allows the wearer of the eyewear to maintain intermediate vision and distance vision unaltered. Constructing an eyewear pair of this type, i.e. with a purely analogue monocle, involves the use of very expensive and complex optical systems and, in the case of customisation on the user, as is the case for the "Through the Lens" type, the same production process must be specifically structured, in order to obtain an eyewear pair suitable for the unique and peculiar characteristics of users' eyes.

Magnifier eyewear with analogue monocles is also typically mono-focal and mono-magnifying. The mono-focality of the system is linked to the fact that said eyewear is used in the performance of delicate operations in which the user is perfectly immobile at a working distance, which he/she considers comfortable. The eyewear is, then, usually mono-magnifying because, according to the type of operation, as well as the subjective operating methods and habits of the user, a magnification value of the optical system is established which is the one sufficient for the performance of all activities by the individual user. Recently, however, the need for alternative solutions with variable magnification monocles and partially variable focus systems has begun to be felt. At the same time, the need is emerging today with increasing insistence to provide magnifying solutions which, in addition to carrying out their main function, which is that in fact of magnification, are in some way able to interface with media for recording the operations carried out by the user.

For this reason, magnifier eyewear solutions equipped with digital screens connected to cameras fixed, directly or indirectly, on the frame of the eyewear have started to spread on the market. Some examples of these solutions can be found in the prior art documents US20150123880A1, EP1884819A1 and WO201102209A1. The US application US20150123880A1 and the European application EP1884819A1 both describe magnifier eyewear for medical use, in which traditional analogue magnifier eyewear is replaced by two video cameras and two digital screens. The video cameras and screens are not integrated into a single device. The screens are, in fact, positioned separately from the video cameras and connected to the frame in US20150123880A1 and to the carrier optics in EP1884819A1.

In these solutions and, in particular, in US20150123880A1, the cameras are in an external position with respect to the rods of the frame with the consequent and inevitable generation of parallax errors with respect to the human eye.

In the international application WO201102209 the analogue magnifier monocles are replaced by monocles comprising, in their interior, both a digital screen and a video camera. These, although integrated into a single monocle, do not communicate directly for the purpose of transmitting the acquired image, but via an external electronic unit. The video camera and the screen are, in fact, connected, respectively, to a first and a second electronic card which, in turn, are connected to an electronic unit external to the monocle. The transmission of the acquired images from the camera to the screen, therefore, takes place via this external electronic unit.

### OBJECTS AND SUMMARY OF THE INVENTION

As mentioned above, the well-known solutions of eyewear with digital magnifiers, described above, while overcoming the limitations of analogue magnifier eyewear, are equipped with a screen and a camera that are not integrated into a single device or, in any case, not directly connected. The lack of integration with direct interfacing between camera and screen results in a delay between the magnified image acquired by the video camera and the image displayed on the screen. In order to guarantee a vision of the magnified image in real time, and that is with a delay not perceivable to the human eye, and, that is, like that which can be obtained with analogue magnifiers, it is necessary for the transmission of the image acquired by the video camera to the digital screen to take place in a time of approximately less than a tenth of a millisecond.

The object of the present invention is, therefore, to provide a magnifier monocle and related eyewear, which overcomes the limitations of analogical magnifiers (i.e. equipped with variable magnification and at least partially variable focus system and allows the recording of the acquired images) and, at the same time, is able to guarantee an enlarged vision of the objects of interest, which is not delayed like current digital magnifier eyewear, but takes place in real time. In other words, the present invention proposes to overcome, at the same time, both the limitations of the current analogical magnifier eyewear and the current digital magnifier eyewear. This object is achieved by the present invention, providing a digital magnifier monocle comprising:
- an eyepiece;
- a lens;
- an organic light-emitting diode screen placed between the eyepiece and the lens;
- a first electronic module, or camera module, for the generation of a digital image, placed between the screen and the lens, referred to as first module, comprising:
   - at least one sensor configured for the transformation of at least one light signal into at least one electrical signal;
   - an electronic system of control of said at least one sensor, said electronic control system being configured for the transformation of said at least one electrical signal into at least one digital image and for the focusing of an object to be displayed with said monocle, said focusing being possible in automatic mode or in a user-controllable mode;
   - a second electronic module placed between the screen and the first electronic module, said second electronic module being divided into:
      - a first unit configured for the transmission of said at least one digital image from the first electronic module to the screen;
      - a second unit configured for the connection to an augmented reality engine and/or the transmission of said at least one digital image from the monocle to a unit external to the monocle, said unit external to the monocle being designed to store said at least one image;
the division of said second electronic module into the first unit and the second unit allowing for the second module to be capable of handling in an independent manner the transmission of image data from the first electronic module to the screen and the transmission of information from the monocle to the unit external to the monocle and from the unit external to the monocle to the monocle.

For the purposes of the present invention, the term 'eyepiece' is understood to indicate an optical system comprising one or more optics placed in the monocle on the user's eye side, and the term 'lens' is understood to indicate an optical system placed in the monocle on the ambient side.

Thanks to the division of the second electronic module into a first and into a second unit, said second module is capable of handling in an independent manner the transmission of image data from the first electronic module (i.e. from the camera) to the screen and the transmission of information from the monocle to the outside, and vice versa. In the case of transmission from the outside to the monocle, this information may be, for example, data to be superimposed on augmented reality. In the case of transmission from the monocle to the outside, however, this information is made up of the same digital images acquired. These images may be sent to the outside for recording on a storage unit and/or for processing with an external image processing unit. Thanks to the independent management of the transmission of image data from the camera to the screen, and of the transmission of data (image and non-image) between monocle and the external unit, it is possible to configure the first unit of the second electronic module in such a way that the transmission of the digital images acquired by the first electronic module, to the screen, takes place in a time of less than a tenth of a millisecond, and thus to obtain a magnified vision of the objects of interest in real time. The main function of the monocle, i.e. the magnifying function, is thus guaranteed without any support from the outside, except possibly the power supply. The latter can moreover be guaranteed also without external energy sources by mounting a battery on the monocle, for example.

At the same time, in addition to the possibility of having a magnified vision in real time, the variability of magnification and focus, typical of digital magnifier eyewear, is in any case guaranteed by the fact that the camera module consists of an integrated circuit with an externally or automatically controllable variable focus system. To this end, therefore, it comprises an electronic system of configured control of focusing and said focusing can be carried out in automatic mode or in a user-controllable mode. The monocle of the present invention is, moreover, designed so that the possibility of having a fixed focus as in analogical magnifiers is maintained. To this end, the user is left the possibility to choose between a focusing mode variable in an automatic manner, typical of digital cameras, and a manual focusing mode that allows the focus to be fixed, obtaining a vision similar to that of analogue magnifiers. The selection, between the two modes, can be made by the user thanks to the second electronic module which, in addition to performing the functions described above, is further configured to co-operate with the camera module in order to manage the focusing mode. In particular, the monocle is equipped with a mode selector element, which allows the interface with the user, making the latter select the focusing mode, said mode selector element being connected to the second electronic module. The latter is able to receive a signal from the selector element, said signal being indicative of the type of mode, automatic or manual, chosen by the user, and to communicate with the system of control of the sensors of the first electronic module, or camera module, so that it effectively varies the focusing mode of the monocle.

A second object of the present invention is, moreover, to avoid the parallax errors that are inevitably present when some solutions of the prior art are used. This object is achieved by the present invention thanks to the integration in the same device (i.e. the magnifier monocle) of the screen and of the camera. In this way, the monocles and, therefore, the camera are positioned exactly in the optical axis of the system and along the line of vision, eliminating any parallax errors at source. Further advantages of the monocle described above are that the first unit of the second electronic module and, more generally, the second electronic module, can be designed using redundancy logics of the electronic paths, in order to minimise the risk of blackout of the system, which would result in a temporary interruption of vision during even very complex operations, such as, for example, surgical ones. Finally, the digital monocle of the present invention can be applied to both "Through the Lens" and "FlipUP" type magnifier eyewear, as if it were a standard analogue monocle in that it can be designed with shape, dimensions and systems of attachment to the frame similar to those of standard monocles.

This and further objects of the present invention will be made clearer by the reading of the following detailed description of some preferred embodiments of the present invention, to be understood purely as a non-limiting example of the more general invention claimed.

### BRIEF DESCRIPTION OF THE DRAWINGS

The following description refers to the accompanying drawings, in which:
- Figure 1 is a schematic representation of a longitudinal section of the monocle of the present invention;
- Figure 2 is a schematic representation of a magnifier eyewear pair seen from above, said eyewear comprising two monocles according to the present invention; and
- Figure 3 is a perspective view of a first embodiment of a magnifier eyewear pair according to the present invention.

### DETAILED DESCRIPTION OF THE INVENTION

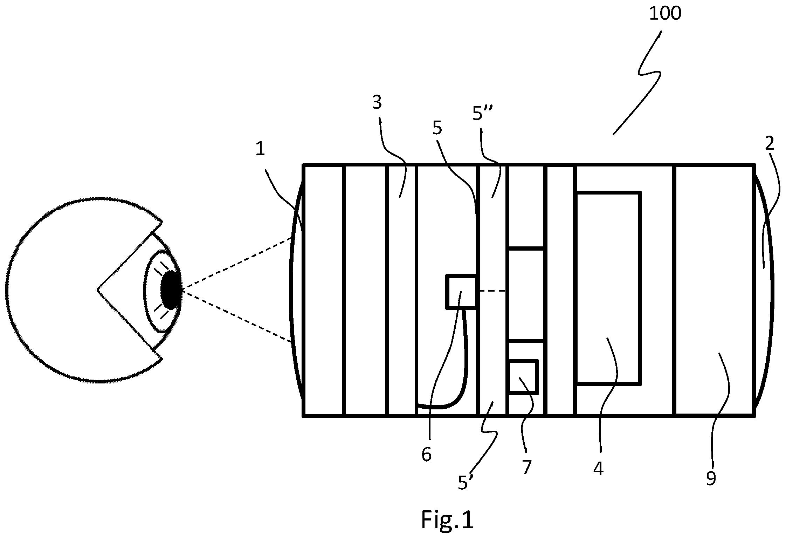

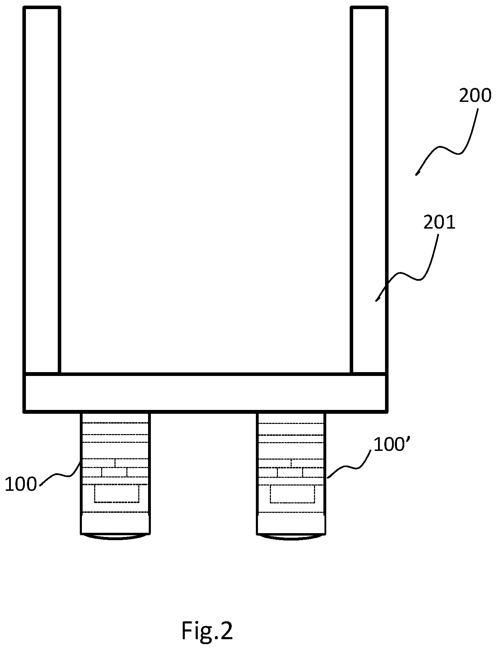

Referring to Figures 1 and 2, a first embodiment of the monocle (100, 100') of the present invention comprises:
- an eyepiece (1);
- a lens (2);
- a screen (3) with organic light-emitting diodes placed between the eyepiece (1) and the lens (2);
- a first electronic module (4), or camera module (4), for the generation of a digital image, placed between the screen (3) and the lens (2), said first module (4) comprising:
   - at least one sensor configured for the transformation of at least one light signal into at least one electrical signal;
   - an electronic system of control of said at least one sensor, said electronic control system being configured for the transformation of said at least one electrical signal into at least one digital image and for the focusing of an object which is to be displayed with said monocle (100, 100'), said focusing being possible in automatic mode or in a user-controllable mode;
   - a second electronic module (5) placed between the screen (3) and the first electronic module (4), said second electronic module (5) being divided into:
      - a first unit (5') configured for the transmission of said at least one digital image from the first electronic module (4) to the screen (3);
      - a second unit (5") configured for the connection with an augmented reality engine and/or the transmission of said at least one digital image from the monocle (100, 100') to a unit external to the monocle (100, 100'), said unit external to the monocle (100, 100') being designed for the storage of said at least one image; the division of said second electronic module (5) into the first unit (5') and the second unit (5") allowing for the second module (5) to be capable of handling in an independent manner the transmission of image data from the first electronic module (4) to the screen (3) and the transmission of information from the monocle (100, 100') to the unit external to the monocle (100, 100') and from the unit external to the monocle (100, 100') to the monocle (100, 100').

The eyepiece (1), which may also consist of a single optic, has the dual function of bringing the image of the screen (3), placed immediately behind the eyepiece (1), to the eye of the user, and of correcting any visual defects of the user. It is possible, in fact, to insert in the monocle (100, 100') of the present invention an eyepiece (1) consisting of one or more optics, capable of making a dioptric correction specific to the recipient of the monocle (100, 100').

The lens (2), instead, serves as an aid to the integrated optics of the first electronic module (4) in order to cover the required working distances and magnifications. In order to optimise in economic terms the production of the monocle (100, 100') of the present invention, it is preferable to vary the integrated optics of the first electronic module (4) as little as possible and to mount the optics of the lens (2) on a standard S-type mounting system (also known in the industry as "S-Mount"). In this way, different optics can be easily disassembled and mounted on the same mounting system. Here it should be noted that the "S-Mount" type mount is a standard mount that uses an M12 male metric thread with a 0.5 mm pitch on the lens and a corresponding female thread on the lens connection.

The first unit (5') of the second electronics module (5) is configured to perform the transmission of said at least one digital image from the first electronics module (4) to the screen (3) in a time less than a tenth of a millisecond. This transmission takes place, preferably, by means of a flat connector (6), whose features depend on the type of screen (3) used. The latter, in a preferred embodiment of the invention, can be a Micro-OLED screen. The second electronic unit (5") of the second electronic module (5) is, instead, equipped with a connector (7) which can be, in turn, connected to an external unit, by means of a connection that can be minimised in number, until arriving at only 3 wires with respect to the 19 of a common HDMI cable typical of those used in Micro-OLED screens, reducing, consequently, the visual impediment generated by the connection itself. The communication with the outside can be carried out, either for the sole purpose of procuring energy for the power supply of the monocle (100, 100'), or for the purpose of sending the acquired images to an external storage and/or processing unit on which to carry out the recording and/or processing of said images, or, further, for the purpose of receiving from an optical augmented reality engine information, graphic and/or text, to be overlaid on the images acquired in real time.

As regards the power supply of the monocle (100, 100'), it is specified here that it can come from a power source external to the monocle (100, 100'), or from a battery mounted on the monocle itself (100, 100'), or, more generally, on the magnifier eyewear (200) to which at least one monocle (100, 100') is attached. In all three cases mentioned above, the second electronic module (5) performs the function of power management of the monocle (100, 100'), i.e. it manages the distribution of the electrical energy between the various components of the monocle (100, 100').

The first electronic module (4), or camera module, can be configured for the acquisition not only of single images, but also of sequences of images, i.e. videos. In this case the second electronic module (5) will be configured to transfer a sequence of images with a frequency of at least 50Hz.

Finally, as mentioned above, the monocle (100, 100') can work both with an automatic and manual focusing mode. For this purpose, the first electronic module (4), or camera module, is equipped with an electronic system for manual or automatic control of focusing. The mode (automatic or manual) of this focusing is, however, selected by the operator by means of an appropriate operator/monocle interface element connected to the second electronic module (5). The latter is able to receive a signal from the interface element, or selector element (not shown in the drawing), said signal being indicative of the type of mode, automatic or manual, chosen by the user. On the basis of the signal received, the second electronic module (5) then communicates with the system of control of the sensors of the first electronic module (4), so that it effectively varies the focusing mode of the monocle (100, 100').

The magnifier monocle (100, 100') described above can be mounted on "FlipUp" type magnifier eyewear comprising:
- a frame;
- a first monocle and a second monocle according to any one of the preceding claims, mounted on said frame.

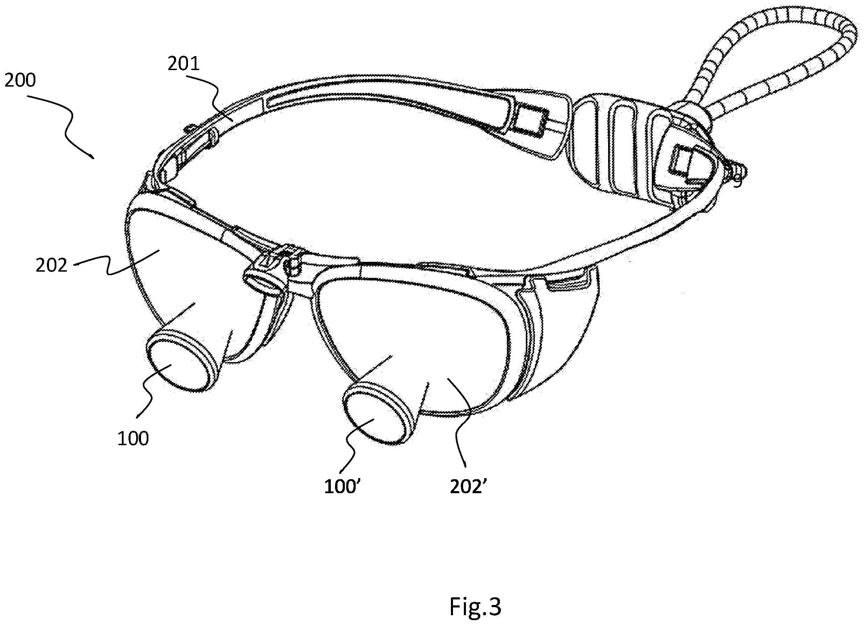

Referring to Figures 2 and 3, the magnifier monocle (100, 100') of the present invention can be mounted on "Through the Lens" type magnifier eyewear, comprising:
- a frame (201);
- a first optic (202) and a second optic (202'); and
- a first monocle (100, 100') according to any one of the preceding claims, said first monocle (100, 100") being mounted on the first optic (202) and a second monocle (100, 100") according to any one of the preceding claims, said second monocle (100, 100") being mounted on the second optic (202').

## Claims

1. Digital magnifier monocle (100, 100') comprising:
• an eyepiece (1);
• a lens (2);
• an organic light emitting diodes screen (3) placed between the eyepiece (1) and the lens (2);
• a first electronic module (4) for the generation of a digital image, placed between the screen (3) and the lens (2), said first module (4) comprising:
- at least one sensor configured for the transformation of at least one light signal into at least one electrical signal;
- an electronic system of control of said at least one sensor, said electronic control system being configured for the transformation of said at least one electrical signal into at least one digital image and for the focusing of an object to be displayed with said monocle (100, 100'), it being possible to perform said focusing in automatic mode or in a mode controllable by a user;
said monocle (100, 100') being **characterised in that** it comprises:
• a second electronic module (5) placed between the screen (3) and the first electronic module (4), said second electronic module (5) being divided into:
- a first unit (5') configured for the transmission of said at least one digital image from the first electronic module (4) to the screen (3); and
- a second unit (5") configured for the connection with an augmented reality motor and/or the transmission of said at least one digital image from the monocle (100, 100') to a unit external to the monocle (100, 100'), said unit external to the monocle (100, 100') being apt to store said at least one image;
the division of said second electronic module (5) into the first unit (5') and the second unit (5") allowing for the second module (5) to be capable of handling in an independent manner the transmission of image data from the first electronic module (4) to the screen (3) and the transmission of information from the monocle (100, 100') to the unit external to the monocle (100, 100') and from the unit external to the monocle (100, 100') to the monocle (100, 100').

2. Monocle (100, 100') according to the preceding claim, wherein the first unit (5') of the second electronic module (5) is configured to perform the transmission of said at least one digital image in a time shorter than a tenth of a millisecond.

3. Monocle (100, 100') according to any one of the preceding claims, wherein the second electronic module (5) is further configured for the management of the power supply of the monocle (100, 100'), said power supply coming from a source of energy external to the monocle (100, 100').

4. Monocle (100, 100') according to claim 1 or 2, wherein the second electronic module (5) is further configured for the management of the power supply of the monocle (100, 100'), said power supply coming from a battery mounted on the monocle (100, 100').

5. Monocle (100, 100') according to any one of the preceding claims, wherein the second electronic module (5) is configured to transfer a sequence of images with a frequency of at least 50Hz.

6. Monocle (100, 100') according to any one of the preceding claims, wherein the eyepiece (1) comprises at least one optic for the correction of dioptric defects.

7. Monocle (100, 100') according to any one of the preceding claims, wherein the lens (2) comprises a system of lens mounting of the S type (9) and at least one optic mounted on said system (9) of optics mounting.

8. Monocle (100, 100') according to any one of the preceding claims, wherein the second electronic module (5) is configured to co-operate with the electronic control system of the first electronic module (4) for the management of the focusing mode.

9. Monocle (100, 100') according to any one of the preceding claims, comprising at least one inertial sensor chosen from among: accelerometer, gyroscope and magnetometer.

10. Magnifier eyewear comprising:
• a frame;
• a first monocle and a second monocle according to any one of the preceding claims, mounted on said frame.

11. Magnifier eyewear (200) comprising:
• a frame (201);
• a first optic (202) and a second optic (202'); and
• a first monocle (100, 100') according to any one of the preceding claims, said first monocle (100, 100'") being mounted on the first optic (202) and a second monocle (100, 100") according to any one of the preceding claims, said second monocle (100, 100") being mounted on the second optic (202').

## Patentansprüche

1. Digitales Lupenmonokel (100, 100'), umfassend:
• ein Okular (1);
• eine Linse (2);
• ein organisches LED-Display (3), das zwischen dem Okular (1) und der Linse (2) angeordnet ist;
• ein erstes elektronisches Modul (4) für die Erzeugung eines digitalen Bildes, das zwischen dem Display (3) und der Linse (2) angeordnet ist, wobei das besagte erste Modul (4) Folgendes umfasst:
- mindestens einen Sensor, der für die Umwandlung mindestens eines Lichtsignals in mindestens ein elektrisches Signal konfiguriert ist;
- ein elektronisches Steuersystem für den besagten mindestens einen Sensor, wobei das besagte elektronische Steuersystem für die Umwandlung des besagten mindestens einen elektrischen Signals in mindestens ein digitales Bild und für die Fokussierung eines Objekts konfiguriert ist, das mit dem besagten Monokel (100, 100') angezeigt werden soll, wobei es möglich ist, die besagte Fokussierung im automatischen Modus oder in einem von einem Benutzer steuerbaren Modus durchzuführen;
wobei das besagte Monokel (100, 100') **dadurch gekennzeichnet ist, dass** es Folgendes umfasst:
• ein zweites elektronisches Modul (5), das zwischen dem Display (3) und dem ersten elektronischen Modul (4) angeordnet ist, wobei das besagte zweite elektronische Modul (5) unterteilt ist in:
- eine erste Einheit (5'), die für die Übertragung des besagten mindestens einen digitalen Bildes von dem ersten elektronischen Modul (4) auf das Display (3) konfiguriert ist; und
- eine zweite Einheit (5"), die für die Verbindung mit einem Augmented-Reality-Motor und/oder die Übertragung des besagten mindestens einen digitalen Bildes von dem Monokel (100, 100') zu einer Einheit außerhalb des Monokels (100, 100') konfiguriert ist, wobei die besagte Einheit außerhalb des Monokels (100, 100') geeignet ist, das besagte mindestens eine Bild zu speichern;
die Unterteilung des besagten zweiten elektronischen Moduls (5) in die erste Einheit (5') und die zweite Einheit (5"), die es dem zweiten Modul (5) ermöglicht, die Übertragung von Bilddaten vom ersten elektronischen Modul (4) an das Display (3) und die Übertragung von Informationen vom Monokel (100, 100') an die Einheit außerhalb des Monokels (100, 100') und von der Einheit außerhalb des Monokels (100, 100') an den Monokel (100, 100') unabhängig zu handhaben.

2. Monokel (100, 100') gemäß dem vorhergehenden Anspruch, wobei die erste Einheit (5') des zweiten elektronischen Moduls (5) konfiguriert ist, um die Übertragung des besagten mindestens einen digitalen Bildes in einer Zeit von weniger als einer Zehntel Millisekunde durchzuführen.

3. Monokel (100, 100') gemäß einem jeden der vorhergehenden Ansprüche, wobei das zweite elektronische Modul (5) ferner für die Verwaltung der Stromversorgung des Monokels (100, 100') konfiguriert ist, wobei die besagte Stromversorgung von einer Energiequelle außerhalb des Monokels (100, 100') herkommt.

4. Monokel (100, 100') gemäß Anspruch 1 oder 2, wobei das zweite elektronische Modul (5) ferner für die Verwaltung der Stromversorgung des Monokels (100, 100') konfiguriert ist, wobei die besagte Stromversorgung von einer Batterie herkommt, die auf dem Monokel (100, 100') montiert ist.

5. Monokel (100, 100') gemäß einem jeden der vorhergehenden Ansprüche, wobei das zweite elektronische Modul (5) konfiguriert ist, um eine Bildsequenz mit einer Frequenz von mindestens 50 Hz zu übertragen.

6. Monokel (100, 100') gemäß einem jeden der vorhergehenden Ansprüche, wobei das Okular (1) mindestens eine Optik zur Korrektur von dioptrischen Fehlern aufweist.

7. Monokel (100, 100') gemäß einem jeden der vorhergehenden Ansprüche, wobei die Linse (2) ein Linsenbefestigungssystem des Typs S (9) und mindestens eine Optik umfasst, die auf dem besagten Optikbefestigungssystem (9) montiert ist.

8. Monokel (100, 100') gemäß einem jeden der vorhergehenden Ansprüche, wobei das zweite elektronische Modul (5) konfiguriert ist, um mit dem elektronischen Steuersystem des ersten elektronischen Moduls (4) für die Verwaltung des Fokussierungsmodus zusammenzuwirken.

9. Monokel (100,100') gemäß einem jeden der vorhergehenden Ansprüche, umfassend mindestens einen Trägheitssensor, ausgewählt aus: Beschleunigungsmesser, Gyroskop und Magnetometer.

10. Lupenbrille, umfassend:
• einen Rahmen;
• ein erstes Monokel und ein zweites Monokel gemäß einem jeden der vorhergehenden Ansprüche, die an dem besagten Rahmen montiert sind.

11. Lupenbrille (200), umfassend:
• einen Rahmen (201);
• eine erste Optik (202) und eine zweite Optik (202'); und
• ein erstes Monokel (100, 100') gemäß einem jeden der vorhergehenden Ansprüche, wobei das besagte erste Monokel (100, 100'") auf der ersten Optik (202) montiert ist, und ein zweites Monokel (100, 100") gemäß einem jeden der vorhergehenden Ansprüche, wobei das besagte zweite Monokel (100, 100") auf der zweiten Optik (202') montiert ist.

## Revendications

1. Monocle à loupe numérique (100, 100') comprenant :
• un oculaire (1) ;
• une lentille (2) ;
• un écran à diodes électroluminescentes organiques (3) placé entre l'oculaire (1) et la lentille (2) ;
• un premier module électronique (4) pour la génération d'une image numérique, placé entre l'écran (3) et la lentille (2), ledit premier module (4) comprenant :
- au moins un capteur configuré pour la transformation d'au moins un signal lumineux en au moins un signal électrique ;
- un système électronique de contrôle dudit capteur au minimum, ledit système électronique de contrôle étant configuré pour la transformation dudit signal électrique au minimum en au moins une image numérique et pour la mise au point d'un objet à afficher avec ledit monocle (100, 100'), cette mise au point pouvant être effectuée en mode automatique ou en mode contrôlable par un utilisateur ;
ledit monocle (100, 100') étant **caractérisé par le fait qu'**il comprend :
• un second module électronique (5) placé entre l'écran (3) et le premier module électronique (4), ledit second module électronique (5) comprenant :
- une première unité (5') configurée pour la transmission de ladite image numérique au minimum du premier module électronique (4) vers l'écran (3) ; et
- une seconde unité (5") configurée pour la connexion avec un moteur de réalité augmentée et/ou la transmission de ladite image numérique au minimum du monocle (100, 100') à une unité externe au monocle (100, 100'), ladite unité externe au monocle (100, 100') étant apte à stocker ladite image au minimum.
la division dudit second module électronique (5) en première unité (5') et seconde unité (5") permettant au second module (5) d'être capable de gérer de manière indépendante la transmission des données d'image du premier module électronique (4) vers l'écran (3) et la transmission des informations du monocle (100, 100') vers l'unité externe au monocle (100, 100') et de l'unité externe au monocle (100, 100') vers le monocle (100, 100').

2. Monocle (100, 100') selon la revendication précédente, où la première unité (5') du second module électronique (5) est configurée pour effectuer la transmission de ladite image numérique au minimum dans un temps inférieur à un dixième de milliseconde.

3. Monocle (100, 100') selon l'une des revendications précédentes, où le second module électronique (5) est en outre configuré pour la gestion de l'alimentation électrique du monocle (100, 100'), ladite alimentation électrique provenant d'une source d'énergie externe au monocle (100, 100').

4. Monocle (100, 100') selon la revendication 1 ou 2, où le second module électronique (5) est en outre configuré pour la gestion de l'alimentation électrique du monocle (100, 100'), ladite alimentation électrique provenant d'une batterie montée sur le monocle (100, 100').

5. Monocle (100, 100') selon l'une des revendications précédentes, où le second module électronique (5) est configuré pour transférer une séquence d'images avec une fréquence d'au moins 50 Hz.

6. Monocle (100, 100') selon l'une des revendications précédentes, où l'oculaire (1) comprend au moins une optique pour la correction des défauts dioptriques.

7. Monocle (100, 100') selon l'une des revendications précédentes, où la lentille (2) comprend un système de montage de lentilles de type S (9) et au moins une optique montée sur ledit système (9) de montage d'optiques.

8. Monocle (100, 100') selon l'une des revendications précédentes, où le second module électronique (5) est configuré pour coopérer avec le système de contrôle électronique du premier module électronique (4) pour la gestion du mode de focalisation.

9. Monocle (100, 100') selon l'une des revendications précédentes, comprenant au moins un capteur inertiel choisi parmi : un accéléromètre, un gyroscope et un magnétomètre.

10. Lunettes à loupe comprenant :
• une monture ;
• un premier monocle et un second monocle selon l'une des revendications précédentes, montés sur ladite monture.

11. Lunettes à loupe (200) comprenant :
• une monture (201) ;
• une première optique (202) et une seconde optique (202') ; et
• un premier monocle (100, 100') selon l'une des revendications précédentes, ledit premier monocle (100, 100') étant monté sur la première optique (202) et un second monocle (100, 100") selon l'une des revendications précédentes, ledit second monocle (100, 100") étant monté sur la seconde optique (202').
